(19) **Europäisches Patentamt**
**European Patent Office**
**Office européen des brevets**

(11) **EP 1 280 768 B1**

(12) **EUROPÄISCHE PATENTSCHRIFT**

(45) Veröffentlichungstag und Bekanntmachung des Hinweises auf die Patenterteilung:
**09.12.2009 Patentblatt 2009/50**

(21) Anmeldenummer: **01951465.2**

(22) Anmeldetag: **30.04.2001**

(51) Int Cl.:
***C07D 201/08*** *(2006.01)* ***C07D 201/16*** *(2006.01)*

(86) Internationale Anmeldenummer:
**PCT/EP2001/004834**

(87) Internationale Veröffentlichungsnummer:
**WO 2001/083442 (08.11.2001 Gazette 2001/45)**

(54) **VERFAHREN ZUR HERSTELLUNG VON CAPROLACTAM AUS 6-AMINOCAPRONITRIL**

METHOD FOR PRODUCING CAPROLACTAM FROM 6-AMINOCAPRONITRILE

PROCEDE DE PREPARATION DE CAPROLACTAME A PARTIR DE 6-AMINOCAPRONITRILE

(84) Benannte Vertragsstaaten:
**AT BE CH CY DE DK ES FI FR GB GR IE IT LI LU MC NL PT SE TR**

(30) Priorität: **03.05.2000 DE 10021199**

(43) Veröffentlichungstag der Anmeldung:
**05.02.2003 Patentblatt 2003/06**

(73) Patentinhaber: **BASF SE**
**67056 Ludwigshafen (DE)**

(72) Erfinder:
• **BASSLER, Peter**
**68519 Viernheim (DE)**
• **BAUMANN, Dieter**
**69190 Walldorf (DE)**
• **FISCHER, Rolf-Hartmuth**
**69121 Heidelberg (DE)**
• **FUCHS, Eberhard**
**67227 Frankenthal (DE)**
• **MELDER, Johann-Peter**
**67459 Böhl-Iggelheim (DE)**
• **OHLBACH, Frank**
**40219 Düsseldorf (DE)**

(56) Entgegenhaltungen:
**EP-A- 0 943 608 WO-A-96/20923**
**WO-A-97/30028**

• **KIRK-OTHMER: "Encyclopedia of Chemical Technology" 1992, WILEY * Seite 827 - Seite 839 ***
• **WINNACKER, KÜCHLER: "Chemische Technology" 1984, HANSER * Seite 218 - Seite 219 ***

EP 1 280 768 B1

**Beschreibung**

Die vorliegende Erfindung betrifft ein Verfahren zur Herstellung von Caprolactam, dadurch gekennzeichnet, dass man

a) eine Mischung (I) enthaltend 6-Aminocapronitril ("ACN") und Wasser in der Flüssigphase zu einer Mischung (II) enthaltend Caprolactam, Ammoniak, Wasser, Hochsieder und Leichtsieder umsetzt in Gegenwart eines Katalysators, anschließend

b) aus Mischung (II) Ammoniak entfernt unter Erhalt einer Mischung (III) enthaltend Caprolactam, Wasser, Hochsieder und Leichtsieder, anschließend

c) aus Mischung (III) Wasser entfernt unter Erhalt einer Mischung (IV) enthaltend Caprolactam, Hochsieder mit einem Siedepunkt über dem von Caprolactam und Leichtsieder mit einem Siedepunkt unter dem von Caprolactam, und zwischen den Schritten c) und d) die Leichtsieder und Hochsieder durch Destillation abtrennt, und anschließend

d) aus Mischung (IV) durch Kristallisation einen Caprolactam enthaltenden Feststoff (V) erhält, wobei der Gewichtsanteil an Caprolactam in Feststoff (V) größer ist als in Mischung (IV) und wobei man die Kristallisation in Abwesenheit von organischen oder anorganischen flüssigen Verdünnungsmitteln durchführt.

Verfahren zur Herstellung von Caprolactam sind allgemein bekannt.

Es ist ebenfalls, beispielsweise aus Ullmann's Encyclopedia of Industrial Chemistry, 5. Ed., Vol. A5, VCH Verlagsgesellschaft mbH, Weinheim (Deutschland), 1986, Seite 46-48, oder Kirk-Othmer, Encyclopedia of Chemical Technology, 4. Ed., Vol. 4, John Wiley & Sons, New York, 1992, Seite 836, allgemein bekannt, dass Caprolactam, das für die Herstellung von Polymeren verwendet wird, eine Reinheit von 99,9 bis 99,94 % aufweisen muss, wobei die Hauptverunreinigung üblicherweise Wasser in einer Menge von 0,04 bis 0,1 % ist. Andere Verunreinigungen dürfen nur im Bereich von maximal wenigen ppm enthalten sein.

So kann Caprolactam durch Beckmann-Umlagerung von Cyclohexanonoxim mit Schwefelsäure oder Oleum hergestellt werden. Nach Neutralisation des auf diese Weise erhaltenen Gemischs mit Ammoniak kann das Caprolactam von dem als Nebenprodukt enstandenen Ammoniumsulfat durch Extraktion mit einem organischen Lösungsmittel erhalten werden.

In Abhängigkeit von den Verfahren zur Herstellung der zur Darstellung des Cyclohexanonoxims eingesetzten Edukte, wie Cyclohexanon und Hydroxylammoniumsulfat, den Oximierungs- und Umlagerungsbedingungen enthält das rohe Caprolactam, das durch Beckmann-Umlagerung erhalten wurde, Verunreinigungen, die sich in Art und Umfang unterscheiden. Typische Verunreinigungen von rohem Caprolactam, das durch Beckmann-Umlagerung hergestellt wurde, sind C-Methylcaprolactame, 6-Methylvalerolactam und n-Pentylacetamid.

Zur Reinigung des bei der Beckmann-Umlagerung erhaltenen Roh-Caprolactams sind verschiedene Verfahren beschrieben.

Gemäß DE-A-1253716 kann das Roh-Caprolactam durch Hydrierung in Suspension in Gegenwart eines Katalysators und unter Zusatz einer Säure gereinigt werden.

Gemäß DE-A-1253716 kann das Roh-Caprolactam durch Hydrierung in Suspension in Gegenwart eines Katalysators und unter Zusatz einer Base gereinigt werden.

DD-A-75083 beschreibt ein Verfahren zur Reinigung von Roh-Caprolactam, in dem das Roh-Caprolactam zunächst destilliert und anschließend, gelöst in einem organischen Lösungsmittel, in Gegenwart eines Katalysators hydriert und dann mit einem Ionentauscher behandelt wird.

Gemäß EP-A-411455 können die charakteristischen wichtigen Qualitätsmerkmale für Caprolactam eingehalten werden, indem man das Roh-Caprolactam kontinuierlich in einem Flüssigphasen-Verfahren hydriert.

Roh-Caprolactam, das durch Hydroformylierung von 3-Pentensäure und/oder ihren Estern zu 5-Formylvaleriansäure(estern) als Hauptprodukten und 4- und 3-Formylvaleriansäure(estern) als Nebenprodukten, extraktiver (WO 97/02228) oder destillativer (WO 97/06126) Abtrennung dieser verzweigten Formylvaleriansäure(ester), aminierender Hydrierung von 5-Formylvaleriansäure(estern) zu 6-Aminocapronsäure(estern) und/oder 6-Aminocapronsäureamid und Cyclisierung von 6-Aminocapronsäure(estern) oder 6-Aminocapronsäureamid erhalten wird, enthält andere typische Verunreinigungen.

So ist beispielsweise aus WO 99/48867, Beispiel 1, bekannt, ausgehend von 5-Formylvaleriansäureestern, nach WO 98/37063, Beispiel 9 aus Gemischen aus 6-Aminocapronsäure, 6-Aminocapronsäureamid und entsprechenden Oligomeren erhaltenes Rohcaprolactam unter Zusatz von 10 Gew.-% Wasser, zu kristallisieren. In diesem Rohcaprolactam, aus dem Hoch- und Leichtsieder vor der Kristallisation nicht abgetrennt wurden, waren 6345 ppm N-Methylcaprolactam, 100 ppm 5-Methylvalerolactam, 78 ppm Valeramid und andere Verunreinigungen enthalten. Die Rohcaprolactam/Wasser-Schmelze wurde bei 50°C homogenisiert und dann auf 30°C abgekühlt. Die ausgefallenen Kristalle wurden abfiltriert und 2 bis 3 Mal mit wässrigem Caprolactam gewaschen. 5-Methylvalerolactam und Valeramid wurden auf 1 ppm, N-Methylcaprolactam auf 51 ppm abgereichert. Aus 73,6 g Rohlactam wurden 33,7 g Reinlactam erhalten (Caprolactam-Ausbeute: 45,8 %). Die Kennzahl der flüchtigen Basen (VB) wurde erst durch eine zweite Kristallisation erreicht. Wurden nach WO 99/48867, Beispiel 3, aus dem Rohcaprolactam vor der Kristallisation Hoch- und Leichtsieder abgetrennt, so betrug die Caprolactam-Ausbeute nach der Kristallisation 52 %.

Aus WO 99/65873 ist weiterhin bekannt, Caprolactam aus Gemischen mit 4-Ethyl-2-pyrrolidon, 5-Methyl-2-piperidon, 3-Ethyl-2-pyrrolidon und 3-Methyl-2-Piperidon oder Octahydrophenazin an Adsorptionsmitteln wie aktivierte Aktivkohle, Molekularsieben oder Zeolithen selektiv zu adsorbieren und nach Desorption reines Caprolactam zu erhalten. An diese Caprolactam-Abtrennung kann sich eine Schmelzkristallisation oder eine Kristallisation aus einem Lösungsmittel anschließen.

Es ist weiterhin bekannt, Roh-Caprolactam durch Kristallisation zu reinigen, das ausgehend von 6-Aminocapronitril nach WO 98/37063, Anspruch 8, zunächst mit Wasser zu 6-Aminocapronsäure hydrolysiert wird. Dann werden Wasser und durch Hydrolyse gebildeter Ammoniak abgetrennt, die gebildete 6-Aminocapronsäure wird cyclisiert und das dabei anfallende Roh-Caprolactam nach WO 99/48867 kristallisiert.

Caprolactam kann auch erhalten werden durch Reaktion von ACN mit Wasser in der Flüssigphase in der Gegenwart oder Abwesenheit eines Katalysators unter Freisetzung von Ammoniak.

Die bei dieser Reaktion erhaltene Mischung enthält neben Caprolactam, Wasser, Ammoniak, gegebenenfalls weiterem flüssigem Verdünnungsmittel Verunreinigungen mit einem Siedepunkt über dem von Caprolactam ("Hochsieder") und solche mit einem Siedepunkt unter dem von Caprolactam ("Leichtsieder").

Aus US-A-496,941, Beispiel, ist bekannt, daß nach der Abtrennung von Wasser, Lösungsmittel, Ammoniak, Leichtsieder und Hochsieder aus einer Mischung, erhalten bei der Umsetzung von ACN mit Wasser und Lösungsmittel, ein rohes Caprolactam mit einer Reinheit von 99,5 % erhalten wird.

Für ein Roh-Caprolactam, das aus ACN in der Flüssigphase erhalten wurde, sind andere Reinigungsverfahren beschrieben, da sich die Verunreinigungen eines solchen Roh-Caprolactams von denen eines Roh-Caprolactams, das durch andere Verfahren erhalten wurde, wie in US-A-5,496,941 beschrieben, deutlich unterscheiden.

Gemäß US-A-5,496,941 wird ACN in einem ersten Schritt in der Flüssigphase zu Caprolactam umgesetzt, Leichtsieder, Wasser, Ammoniak und gegebenenfalls weitere Lösungsmittel gleichzeitig abgetrennt, Hochsieder abgetrennt unter Erhalt eines Roh-Caprolactams in einer Reinheit von 99,5 %, dieses Roh-Caprolactam in Gegenwart eines Katalysators hydriert, das erhaltene Produkt mit einem sauren Ionentauscher oder Schwefelsäure behandelt und das erhaltene Produkt in Gegenwart einer Base destilliert.

Aus WO 96/20923 ist ein Verfahren zur Reinigung von Rohcaprolactam bekannt, das aus der Flüssigphasen-Cyclisierung von 6-Aminocapronitril mit Wasser in Gegenwart eines Lösungsmittels und von heterogenen Katalysatoren stammt. Hierbei wird Rohcaprolactam zunächst hydriert, dann mit sauren Agenzien behandelt und zuletzt in Gegenwart von Alkali destilliert. Nachteilig an diesem Reinigungsverfahren ist, dass drei separate Reaktionsschritte für die Herstellung von Rein-Caprolactam benötigt werden.

Die genannten Verfahren zur Reinigung von Roh-Caprolactam, das aus ACN hergestellt wurde, weisen den Nachteil auf, daß sie technisch aufwendig und energieintensiv, insbesondere durch die zahlreichen Trennschritte, sind.

Der vorliegenden Erfindung lag die Aufgabe zugrunde, ein Verfahren zur Verfügung zu stellen, das die Herstellung von Caprolactam, das in der Flüssigphase ausgehend von ACN erhalten wurde, in hoher Reinheit auf technisch einfache und energiesparende Weise ermöglicht.

Demgemäß wurde das eingangs definierte Verfahren gefunden.

Gemäß Schritt a) wird eine Mischung (I) enthaltend 6-Aminocapronitril, Wasser und gegebenenfalls flüssiges Verdünnungsmittel zu einer Mischung (II) enthaltend Caprolactam, Ammoniak, Wasser, gegebenenfalls flüssiges Verdünnungsmittel, Hochsieder und Leichtsieder in Gegenwart eines die Umsetzung katalytisch fördernden Feststoffes in der Flüssigphase umgesetzt.

Das für Schritt a) erforderliche ACN kann, wie aus Ullmann's Encyclopedia of Industrial Chemistry, 5. Ed., Vol. A5, VCH Verlagsgesellschaft mbH, Weinheim (Deutschland), 1986, Seite 46, Fig. 8, allgemein bekannt, aus Adipodinitril erhalten werden.

Besonders in Betracht kommt dabei die partielle katalytische Hydrierung von Adipodinitril in Gegenwart von Ammoniak als Lösungsmittel und beispielsweise als Suspensionskatalysator Rhodium auf Magnesiumoxid (US-A-4,601,859), Raney Nickel (US-A-2,762,835, WO 92/21650), Nickel auf Aluminiumoxid (US-A-2,208,598) oder als Festbettkatalysator Cu-Co-Zn-Spinell (DE-B-954416, US-A-2,257,814) oder Eisen (DE-A-42 35 466) oder ein Verfahren gemäß US-A-2,245,129, US-A-2,301,964, EP-A-150295, FR-A-2 029 540 oder einem in US-A-5,496,941 beschriebenen Verfahren.

Das für diese Umsetzung erforderliche Adipodinitril wird technisch hergestellt, beispielsweise durch doppelte Hydrocyanierung von Butadien in Gegenwart von Nickel enthaltenden Katalysatoren, und ist kommerziell beispielsweise über die Firma Aldrich-Chemie Gesellschaft mbH & Co. KG, Steinheim, Deutschland verfügbar.

Die Umsetzung von Mischung (I) zu Mischung (II) kann gemäß FR-A-2029540 in Gegenwart von Katalysatoren durchgeführt werden, wobei als Katalysatoren metallisches Zn oder Cu-Pulver oder Oxide, Hydroxide, Halogenide, Cyanide des Rubidiums, Bleis, Quecksilbers oder der Elemente mit einer Ordnungszahl 21 bis 30 oder 39 bis 48 Verwendung finden. Die beschriebenen Katalysatoren werden in diskontinuierlich betriebenen Rührautoklaven als Suspensionskatalysatoren eingesetzt.

Als besonders bevorzugt kommt als Schritt a) ein Verfahren in Betracht, wie es in US-A-5,646,277, US-A-5,739,324 oder WO 59/14665 beschrieben ist.

Bei diesen Verfahren wird die Umsetzung in flüssiger Phase bei Temperaturen von im allgemeinen 140 bis 320°C, vorzugsweise 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.

Die Umsetzung kann diskontinuierlich, vorzugsweise kontinuierlich durchgeführt werden. Als Reaktor kommt eine Rührkessel, Autoklav, vorzugsweise ein Festbett-Röhrenreaktor, in Betracht. Es sind auch Kombinationen solcher Reaktoren möglich.

Pro mol ACN werden im allgemeinen mindestens 0,1 mol, vorzugsweise 0,5 bis 100 und insbesondere 1 bis 20 mol Wasser eingesetzt.

Vorteilhaft wird das ACN in Form einer 1 bis 50 gew.-%igen, insbesondere 5 bis 50 gew.-%igen, besonders vorzugsweise 5 bis 30 gew.-%igen Lösung in Wasser (wobei dann das Lösungsmittel gleichzeitig Reaktionspartner ist) oder in Gemischen enthaltend Wasser und ein flüssiges Verdünnungsmittel eingesetzt. Als flüssiges Verdünnungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer flüssiger Verdünnungsmittel Anwendung finden. Mischungen aus Wasser und Alkanolen im Gewichtsverhältnis Wasser/Alkanol 1-75/25-99, vorzugsweise 1-50/50-99 haben sich in einigen Fällen als besonders vorteilhaft herausgestellt.

Es ist prinzipiell genauso möglich, ACN als Reaktand und gleichzeitig Lösungsmittel anzuwenden.

Als heterogene Katalysatoren können beispielsweise verwendet werden: Saure, basische oder amphotere Oxide der Elemente der zweiten, dritten oder vierten Hauptgruppe des Periodensystems, wie Calciumoxid, Magnesiumoxid, Boroxid, Aluminiumoxid, Zinn-Oxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titanoxid, amorph, als Anatas oder Rutil, Zirkonoxid, zinkoxid, Manganoxid oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:

Vanadiniumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin sind Zeolithe, Phosphate und Heteropolysäuren, sowie saure und alkalische Ionenaustauscher wie beispielsweise Nafion als geeignete Katalysatoren zu nennen.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Als besonders bevorzugte Katalysatoren, die unter den oben beschriebenen Reaktionsbedingungen sehr hohe Umsätze, Ausbeuten, Selektivitäten und Standzeiten besitzen, kommen heterogene Katalysatoren auf Basis Titanoxid, Zirkonoxid, Ceroxid und Aluminiumoxid, insbesondere Titandioxid, in Betracht. Diese können in Form von Pulvern, Gries, Splitt, Strängen oder zu Tabletten gepreßt, verwendet werden. Die Form der Oxide richtet sich in der Regel nach den Erfordernissen der jeweiligen Reaktionsführung, wobei in Suspension Pulver oder Gries verwendet wird. Bei der Festbettfahrweise werden üblicherweise Tabletten oder Stränge mit Durchmessern zwischen 1 mm und 10 mm verwendet.

Aluminiumoxid ist in allen Modifikationen, die durch Erhitzen der Vorläuferverbindungen Aluminiumhydroxid (Gibbsit, Böhmit, Pseudo-Böhmit, Bayerit und Diaspor) bei unterschiedlichen Temperaturen erhalten werden können, geeignet. Dazu gehören insbesondere gamma- und alpha-Aluminiumoxid und deren Gemische.

Die Oxide können in reiner Form (Gehalt des jeweiligen Oxids > 80 Gew.-%), als Gemisch der oben genannten Oxide, wobei die Summe der oben genannten Oxide > 80 Gew.-% betragen soll, oder als Trägerkatalysator, wobei die oben genannten Oxide auf einen mechanisch und chemisch stabilen Träger meist mit hoher Oberfläche aufgebracht werden können, verwendet werden.

Die reinen Oxide können durch Fällung aus wäßrigen Lösungen hergestellt worden sein, z.B. Titandioxid nach dem Sulfatprozeß oder durch andere Verfahren wie die pyrogene Herstellung von feinen Aluminiumoxid-, Titandioxid- oder Zirkondioxid-Pulvern, die käuflich zu erhalten sind.

Zur Herstellung von Gemischen der verschiedenen Oxide stehen mehrere Methoden zur Wahl. Die Oxide oder deren Vorläuferverbindungen, die durch Calzinieren in die Oxide umwandelbar sind, können z.B. durch eine gemeinsame Fällung aus Lösung hergestellt werden. Dabei wird im allgemeinen eine sehr gute Verteilung der beiden verwendeten Oxide erhalten. Die Oxid- oder Vorläufergemische können auch durch eine Fällung des einen Oxids oder Vorläufers in Gegenwart des als Suspension von fein verteilten Teilchen vorliegenden zweiten Oxids oder Vorläufers ausgefällt werden. Eine weitere Methode besteht im mechanischen Mischen der Oxid- oder Vorläuferpulver, wobei dieses Gemisch als Ausgangsmaterial zur Herstellung von Strängen oder Tabletten Verwendung finden kann.

Zur Herstellung von Trägerkatalysatoren bieten sich im Prinzip alle in der Literatur beschriebenen Methoden an. So können die Oxide in Form ihrer Sole durch einfaches Tränken auf dem Träger aufgebracht werden. Durch Trocknen und Calzinieren werden die flüchtigen Bestandteile des Sols üblicherweise aus dem Katalysator entfernt. Solche Sole sind für Titandioxid, Aluminiumoxid und Zirkondioxid käuflich erhältlich.

Eine weitere Möglichkeit zum Aufbringen von Schichten der aktiven Oxide besteht in der Hydrolyse oder Pyrolyse von organischen oder anorganischen Verbindungen. So kann ein keramischer Träger mit Titandioxid durch Hydrolyse von Titan-Isopropylat oder anderen Ti-Alkoxiden in dünner Schicht belegt werden. Weitere geeignete Verbindungen sind unter anderen $TiCl_4$, Zirkonylchlorid, Aluminiumnitrat und Cernitrat. Geeignete Träger sind Pulver, Stränge oder Tabletten der genannten Oxide selbst oder anderer stabiler Oxide wie Siliciumdioxid. Die verwendeten Träger können zur Verbesserung des Stofftransports makroporös ausgestaltet sein.

Gemäß Schritt b) wird aus Mischung (II) Ammoniak entfernt unter Erhalt einer Mischung (III) enthaltend Caprolactam, Wasser, gegebenenfalls flüssiges Verdünnungsmittel, Hochsieder und Leichtsieder.

Die Abtrennung des Ammoniaks aus Mischung (II) kann prinzipiell nach an sich für die Stofftrennung bekannten Verfahren, wie Extraktion oder vorzugsweise Destillation, oder eine Kombination solcher Verfahren erfolgen.

Die Destillation kann man vorteilhaft bei Sumpftemperaturen von 60 bis 220°C, insbesondere von 100 bis 220°C durchführen. Dabei stellt man üblicherweise einen Druck, gemessen am Kopf der Destillationsverrichtung von 2 bis 30 bar absolut ein.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

Gemäß Schritt c) werden aus Mischung (III) Wasser und gegebenenfalls flüssige Verdünnungsmittel entfernt unter Erhalt einer Mischung (IV) enthaltend Caprolactam, Hochsieder und Leichtsieder.

Wurde in Schritt a) ein flüssiges Verdünnungsmittel eingesetzt können Wasser und flüssiges Verdünnungsmittel in Schritt c) gleichzeitig oder das Wasser vor oder nach dem flüssigen Verdünnungsmittel abgetrennt werden.

Die Abtrennung des Wassers aus Mischung (III) kann prinzipiell nach an sich für die Stofftrennung bekannten Verfahren, wie Extraktion, Kristallisation oder vorzugsweise Destillation, oder eine Kombination solcher Verfahren erfolgen.

Die Destillation kann man vorteilhaft bei Sumpftemperaturen von 50 bis 250°C, insbesondere von 100 bis 230°C durchführen.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

Besonders bevorzugt ist eine wärmegekoppelte mehrstufige Abtrennung des Wassers und gegebenenfalls des flüssigen Verdünnungsmittels.

Vor der Zuführung der Mischung (IV) in Schritt d) kommt die Abtrennung von Leichtsieder und Hochsieder, vorteilhaft die Abtrennung nur der Hochsieder, insbesondere weder eine Abtrennung von Leichtsieder noch von Hochsieder, besonders vorteilhaft die Abtrennung nur der Leichtsieder aus der Mischung (IV) in Betracht.

Werden Leichtsieder und Hochsieder aus der Mischung abgetrennt, so können die Leichtsieder vor, nach oder gemeinsam mit den Hochsiedern abgetrennt werden.

Im Falle einer Abtrennung von Leichtsieder und Hochsieder oder nur Hochsieder oder nur Leichtsieder kann die Abtrennung prinzipiell nach an sich für die Stofftrennung bekannten Verfahren, wie Extraktion, Kristallisation oder vorzugsweise Destillation, oder eine Kombination solcher Verfahren verfolgen.

Die Destillation kann man vorteilhaft bei Sumpftemperaturen von 50 bis 250°C, insbesondere von 100 bis 230°C durchführen. Dabei stellt man üblicherweise ein Druck, gemessen am Kopf der Destillationsverrichtung von 1 bis 500, vorzugsweise 5 bis 100 mbar absolut ein.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation zur Abtrennung der Leichtsieder kann man in mehreren, wie 2 oder 3 Kolonnen, vorteilhaft einer einzigen Kolonne durchführen.

Die Destillation zur Abtrennung der Hochsieder kann man in mehreren, wie 2 oder 3 Kolonnen, vorteilhaft einer einzigen Kolonne durchführen.

Gemäß Schritt d) wird aus Mischung (IV) durch partielle Kristallisation ein Caprolactam enthaltender Feststoff (V) erhalten, wobei der Gewichtsanteil an Caprolactam in Feststoff (V) größer ist als in Mischung (IV).

Die Summe der Gehalte an Hochsieder und Leichtsieder, wobei Wasser und organische Verdünnungsmittel nicht mitgerechnet werden, in der in Schritt d) eingesetzten Mischung (IV) beträgt vorteilhaft mindestens 100 Gew.-ppm, vorzugsweise 200 Gew.-ppm, besonders bevorzugt mindestens 500 Gew.-ppm, insbesondere mindestens 1000 Gew.-ppm bezogen auf Mischung (IV).

Die Kristallisation kann diskontinuierlich durchgeführt werden. Die Kristallisation kann kontinuierlich durchgeführt werden.

Die Kristallisation kann mit Zusatz eines Hilfsstoffes, wie eines organischen oder anorganischen flüssigen Verdünnungsmittels, beispielsweise Wasser, vorzugsweise ohne Zusatz eines Hilfsstoffes durchgeführt werden.

Die Kristallisation kann einstufig oder mehrstufig, wie zwei-, drei- oder vierstufig, vorzugsweise einstufig durchgeführt werden. In einer anderen bevorzugten Ausführungsform der Erfindung kann die Kristallisation als fraktionierte Kristallisation durchgeführt werden.

Üblicherweise werden bei fraktionierter Kristallisation alle Stufen, die ein Kristallisat (Caprolactam) erzeugen, das reiner ist als das zugeführte Rohprodukt (Roh-Caprolactam), Reinigungsstufen genannt und alle anderen Stufen Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der verbleibenden flüssigen Phase ("Mutterlauge") abgetrennt wird und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Vorteilhafterweise liegt die Temperatur der Lösung oder Schmelze während der Kristallisation nicht oberhalb des Schmelzpunktes von Caprolactam (70°C), vorzugsweise zwischen -10 und dem Schmelzpunkt von Caprolactam, insbesondere zwischen 20 und dem Schmelzpunkt von Caprolactam. Der Feststoffgehalt im Kristallisator liegt üblicherweise zwischen 0 und 70 g bevorzugt zwischen 30 und 60 g pro 100 g Einsatz.

In einer weiteren vorteilhaften Ausführung der Erfindung erfolgt die Kristallisation in Apparaten, in denen die Kristalle im Kristallisationsapparat an gekühlten Flächen aufwachsen, d.h. im Apparat fixiert sind (z. B. Schichtkristallisationsverfahren der Fa. Sulzer Chemtech (Schweiz) oder Statisches Kristallisationsverfahren der Fa. BEFS PROKEM (Frankreich).

Weiterhin kann die Kristallisation durch Kühlung von Apparatewänden oder durch Verdampfung einer Lösung des Roh-Caprolactams im Vakuum erfolgen. Geeignet sind hierfür besonders 5 bis 30 gew.-%ige Lösungen des Roh-Caprolactams in einem flüssigen Verdünnungsmittel, insbesondere Wasser.

Bei der Kristallisation durch Kühlung kann die Wärme über Kratzkühler, die mit einem Rührkessel oder einem Behälter ohne Rührwerk verbunden sind, abgeführt werden. Der Umlauf der Kristallsuspension kann hierbei durch eine Pumpe gewährleistet werden. Daneben besteht auch die Möglichkeit, die Wärme über die Wand eines Rührkessels mit wandgängigem Rührer abzuführen. Eine weitere bevorzugte Ausführungsform bei der Kühlungskristallisation ist die Verwendung von Kühlscheibenkristallisatoren, wie sie z.B. von der Fa. Gouda (Holland) hergestellt werden. Bei einer weiteren geeigneten Variante zur Kristallisation durch Kühlung kann die Wärme über herkömmliche Wärmeüberträger (bevorzugt Rohrbündel- oder Plattenwärmeüberträger) abgeführt werden. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt üblicherweise die Umschaltung auf einen zweiten Apparat. Während der Betriebszeit des zweiten Apparats kann der erste Apparat regeneriert (vorzugsweise durch Abschmelzen der Kristallschicht oder Durchspülen des Apparats mit ungesättigter Lösung) werden. Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche Verdampfung der Lösung im Vakuum erfolgen.

Zur Abtrennung der Mutterlauge von dem auskristallisierten Caprolactam kommen die an sich bekannten Verfahren der Fest-Flüssig-Trennung in Betracht.

In einer bevorzugten Ausführungsform der Erfindung können die Kristalle durch Filtrieren und/oder Zentrifugieren von der Mutterlauge abgetrennt werden. Vorteilhafterweise kann dem Filtrieren oder Zentrifugieren eine Voreindickung der Suspension, zum Beispiel durch einen oder mehrere Hydrozyklone, vorgeschaltet werden. Zum Zentrifugieren kommen an sich bekannte Zentrifugen, die diskontinuierlich oder kontinuierlich arbeiten, in Betracht. Am vorteilhaftesten können Schubzentrifugen verwendet werden, die ein- oder mehrstufig betrieben werden können. Daneben eignen sich auch Schneckensiebzentrifugen oder Schneckenaustragszentrifugen (Dekanter). Eine Filtration kann vorteilhafterweise mittels Filternutschen erfolgen, die diskontinuierlich oder kontinuierlich, mit oder ohne Rührwerk, oder mittels Bandfilter betrieben werden. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/ oder Schwitzen der Kristalle oder des Kristallkuchens an.

Beim Waschen sollte die Waschflüssigkeitsmenge vorzugsweise zwischen 0 und 500 g Waschflüssigkeit/100 g Kristallisat, vorzugsweise zwischen 30 und 200 g Waschflüssigkeit/100 g kristallisat betragen.

Als Waschflüssigkeit kommen organische oder anorganische Flüssigkeiten oder Gemische solcher Flüssigkeiten in Betracht. Bevorzugte Waschflüssigkeiten sind beispielsweise

a) für den Fall, daß in der Kristallisation in Schritt d) ein flüssiges Verdünnungsmittel eingesetzt wurde, dieses flüssige Verdünnungsmittel,

b) eine Schmelze eines in einer Kristallisationsstufe gemäß Schritt d) erhaltenen Kristallisats,

c) eine in einer Kristallisationsstufe gemäß Schritt d) erhaltene Mutterlauge, oder

d) eine Schmelze eines in einer Kristallisationsstufe gemäß Schritt d) eingesetzten Eduktes.

Das Waschen kann in hierfür üblichen Apparaten erfolgen. Vorteilhafterweise können Waschkolonnen, in denen die Abtrennung der Mutterlauge und das Waschen in einem Apparat erfolgen, Zentrifugen, die ein- oder mehrstufig betrieben werden können, oder Filternutschen oder Bandfilter verwendet werden. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Die Waschflüssigkeit kann, insbesondere bei Kristallisation ohne Zusatz eines Hilfsstoffs, gegebenenfalls nach Abtrennung von Verunreinigungen, in die Kristallisation zurückgeführt werden.

Unter Schwitzen versteht man üblicherweise ein lokales Abschmelzen verunreinigter Bereiche. Vorteilhafterweise sollte die Schwitzmenge 0,1 bis 90 g abgeschmolzenes Kristallisat/100 g Kristallisat vor dem Schwitzen, vorzugsweise 5 bis 35 g abgeschmolzenes Kristallisat/100 g Kristallisat betragen. Besonders bevorzugt ist die Durchführung des Schwitzens auf Zentrifugen oder Bandfiltern. Auch die Durchführung einer Kombination aus Waschen und Schwitzen in einem Apparat kann geeignet sein.

Die Mutterlauge kann, insbesondere bei Kristallisation ohne Zusatz eines Hilfsstoffs, gegebenenfalls nach Abtrennung von Verunreinigungen, in die Kristallisation zurückgeführt werden. Nach dem vorliegenden Verfahren kann Caprolactam in einer Reinheit von mindestens 99,90 Gew.-%, vorzugsweise 99,90 bis 99,99

Gew-%, erhalten werden.

Das nach dem erfindungsgemäßen Verfahren erhältliche Caprolactam kann zur Herstellung von Polyamiden, wie Polycaprolactam, verwendet werden.

Beispiele

Beispiel 1a

Herstellung von Roh-Caprolactam

Die Reinigungsfolge wurde mit Rohcaprolactam durchgeführt, das nach WO 95/14664 durch Cyclisierung einer 10 %igen ethanolischen 6-Aminocapronitril(ACN)-Lösung in Gegenwart von 2,5 Molen Wasser pro Mol ACN erhalten wurde:

In einen geheizten Rohrreaktor von 25 ml Inhalt (Durchmesser 6 mm; Länge 800 mm), der mit Titandioxid (Anatas) in Form von 1,5 mm Strängen gefüllt war, wurde bei 100 bar eine Lösung von 6-Aminocapronsäurenitril (ACN) in Wasser und Ethanol (10 Gew.-% ACN, 4,0 Gew.-% Wasser, Rest: Ethanol) geleitet, wobei die Reaktionstemperatur 245 °C und die Verweilzeit 30 min betrugen. Der den Reaktor verlassende Produktstrom wurde gaschromatographisch analysiert: Umsatz: 100 %, Ausbeute: 87 %.

Der Reaktionsaustrag wurde durch fraktionierende Destillation von Hoch- und Leichtsiedern befreit. Das so erhaltene Rohcaprolactam besaß laut gaschromatographischer Analyse eine Reinheit von 99,90 %.

Beispiel 1b

Reinigung von Rohcaprolactam durch Kristallisation

492 g flüssiges Rohcaprolactam aus Beispiel 1a wurden an der ebenen gekühlten Bodenplatte eines gerührten Behälters zur Kristallisation gebracht.

Die Bodenplatte des Behälters wurde ausgehend von einer Anfangstemperatur von 75°C mit -50 K/h abgekühlt. Dabei bildete sich nach Unterschreiten des Schmelzpunkts der Rohcaprolactamschmelze eine stetig wachsende Kristallschicht aus reinem Caprolactam auf der Kühlfläche. Um einen guten Stoffübergang zwischen der flüssigen und der festen Phase während des Wachstumsprozesses zu gewährleisten, wurde die Rohcaprolactamschmelze mit einem Blattrührer bei 500 Upm gerührt. Nach Erreichen einer Kristallmasse von 378 g (Ausbeute 77 Gew.-%) wurde die Kühlung beendet, der Rührer abgeschaltet und der Rührbehälter um 180° gedreht, so dass nun der Boden nach oben ausgerichtet war. Dadurch wurde die Trennung der an Verunreinigungen angereicherten Restschmelze (Mutterlauge, 23 Gew.-%) von der oben an der Bodenplatte haftenden Caprolactam-Kristallschicht erreicht. Die Caprolactam-Kristallmasse konnte durch Demontage der Bodenplatte des Behälters entnommen werden.

Die Eigenschaften des Rohcaprolactams aus Beispiel 1a, der Kristalle und der Mutterlauge aus Beispiel 1b sind aus Tabelle 1 ersichtlich.

Die übliche erforderliche Spezifikation von kommerziell verfügbarem Caprolactam, sowie die entsprechenden Werte des Rohlactams aus Beispiel 1a und des gemäß Beispiel 1b gereinigten Caprolactams ("Kristalle") sind aus Tabelle 2 ersichtlich.

Tabelle 1

|  | Menge [Gew.-% ] | Capro | ACN | HMD | ACSEE | EECL | ZECL |
|---|---|---|---|---|---|---|---|
| Rohlactam | 100 | 99,90 | 52 ppm | 31 ppm | 101 ppm | 35 ppm | 22 ppm |
| Kristalle | 77 | 99,991 | 4 ppm | < 1 ppm | 5 ppm | 12 ppm | 7 ppm |
| Mutterlauge | 23 | 99,832 | 205 ppm | 122 ppm | 431 ppm | 105 ppm | 81 ppm |

Tabelle 2

|  | Freie Basen [meq/kg] | Flüchtige Basen [meq/ kg] | Extinktion | Farbzahl | PAZ |
|---|---|---|---|---|---|
| Spezifikation | 0,1 | 0,5 | 0,05 | 5 | 4 |
| Rohlactam | 1,3 | 0,73 | 0,18 | 7,2 | 7,8 |
| Kristalle | 0,09 | 0,34 | 0 | 0 | 1,6 |

Abkürzungen:

ACN: 6-Aminocapronitril
HMD: Hexamethylendiamin
ACSEE: 6-Aminocapronsäureethylester
EECL: E-alpha-Ethylidencaprolactam
ZECL: Z-alpha-Ethylidencaprolactam

Analysenmethoden

Freie Basen

Zur Bestimmung der freien Basen wurden 150 ml destilliertes und mit Stickstoff begastes $CO_2$-freies Wasser mit 0,01 N Natronlauge auf genau pH 7,0 eingestellt und 50 +/- 0,1 g Caprolactam hinzugegeben. Anschließend wurde bei 25°C mit 0,01 N Salzsäure auf pH 7,0 titriert. Der Anteil an freier Base ließ sich dann nach untenstehender Formel berechnen, wobei A (m1) den Verbrauch an 0,01 N Salzsäure bedeutet. Freie Basen = 0,01 * A * 1000/50 = 0,2 * A meq/kg.

Flüchtige Basen (FB)

Die flüchtigen Basen wurden nach ISO-Vorschrift 8661 ("Caprolactam for industrial use - Determination of volatile bases content") bestimmt.

Bei einer Destillation im alkalischen Medium wurden die flüchtigen Basen aus der Probe freigesetzt (Kjeldahl-Apparatur), in einer 0,01 N Salzsäure aufgefangen und durch Titration mit 0,01 N Natronlauge bestimmt, die Einwaage betrug 20 $\pm$ 0,1 g Caprolactam.

$$FB = \frac{(B - A)\ x\ 0,01}{20} x\ 1000 \quad meq/kg$$

A = Verbrauch an 0,01 N Natronlauge

B = Verbrauch an 0,01 N Natronlauge für eine Blindbestimmung Extinktion

Die Extinktion wurde gemäß ISO-Vorschrift 7059 ("Caprolactam for Industrial Use - Determination of Absorbance at a Wavelength of 290 nm") durchgeführt.

Farbzahl

Die Farbzahl wurde gemäß ISO-Vorschrift 8112 bestimmt.

50 $\pm$ 0,1 g Caprolactam wurden in einem 250 ml Erlenmeyer-Kolben in 50 ml destilliertem Wasser gelöst und so lange stehengelassen, bis die Luftblasen verschwunden waren. In zwei Vergleichsküvetten (1=5 cm) wurde bei $\lambda$ = 390 nm die Extinktionsdifferenz $E_{290}$ zwischen Wasser und CL/Wasser bestimmt.

$$Farbzahl = 150 * E_{290}\ (auf\ ganze\ Zahl\ gerundet)$$

Die PAZ wurde gemäß ISO-Vorschrift 8660 bestimmt.

Hierzu wurden gleiche Mengen einer 0,01 N Kaliumpermanganat-Lösung zu einer 3 % (m/m) wässrigen Caprolactam-Lösung und zu einer Blindprobe (dest. Wasser) gegeben. Nach 10 Minuten wurden die Extinktionen E bei 420 nm sowohl der Caprolactamprobe als auch der Blindprobe verglichen. Die Permanganatabsorptionszahl wird über folgende Formel ermittelt:

$$PAZ\ (PI) = (E-E_0)_{420} * 100/3$$

**Patentansprüche**

1. Verfahren zur Herstellung von Caprolactam, **dadurch gekennzeichnet, dass** man

a) eine Mischung (I) enthaltend 6-Aminocapronitril und Wasser in der Flüssigphase zu einer Mischung (II) enthaltend Caprolactam, Ammoniak, Wasser, Hochsieder und Leichtsieder umsetzt in Gegenwart eines Katalysators, anschließend

b) aus Mischung (II) Ammoniak entfernt unter Erhalt einer Mischung (III) enthaltend Caprolactam, Wasser, Hochsieder und Leichtsieder, anschließend

c) aus Mischung (III) Wasser entfernt unter Erhalt einer Mischung (IV) enthaltend Caprolactam, Hochsieder mit einem Siedepunkt über dem von Caprolactam und Leichtsieder mit einem Siedepunkt unter dem von Caprolactam, und zwischen den Schritten c) und d) die Leichtsieder und Hochsieder durch Destillation abtrennt, und anschließend

d) aus Mischung (IV) durch Kristallisation einen Caprolactam enthaltenden Feststoff (V) erhält, wobei der Gewichtsanteil an Caprolactam in Feststoff (V) größer ist als in Mischung (IV) und wobei man die Kristallisation in Abwesenheit von organischen oder anorganischen flüssigen Verdünnungsmitteln durchführt.

2. Verfahren nach Anspruch 1, wobei Mischung (I) zusätzlich ein organisches flüssiges Verdünnungsmittel enthält.

**3.** Verfahren nach Anspruch 2, wobei man das flüssige Verdünnungsmittel in Schritt c) vor, während oder nach der Abtrennung des Wassers aus der Mischung (III) entfernt.

**4.** Verfahren nach den Ansprüchen 1 bis 3, wobei man die Kristallisation in Schritt d) an einer gekühlten Fläche durchführt, an der Feststoff (V) aufwächst.

**5.** Verfahren nach den Ansprüchen 1 bis 4, wobei die nach der Kristallisation gemäß Schritt d) erhaltene Mutterlauge mit Mischung (IV) vermischt und in Schritt d) zurückgeführt wird.

**6.** Verfahren nach den Ansprüchen 1 bis 5, wobei man die Kristallisation in Schritt d) diskontinuierlich durchführt.

**Claims**

**1.** A process for the preparation of caprolactam, wherein

a) a mixture (I) comprising 6-aminocapronitrile and water is reacted in the liquid phase, in the presence of a catalyst, to give a mixture (II) comprising caprolactam, ammonia, water, high-boiling components and low-boiling components,
b) ammonia is then removed from the mixture (II) to give a mixture (III) comprising caprolactam, water, high-boiling components and low-boiling components,
c) water is then removed from the mixture (III) to give a mixture (IV) comprising caprolactam, high-boiling components boiling above caprolactam and low-boiling components boiling below caprolactam, and the low-boiling and high-boiling components are separated off by distillation between steps c) and d), and
d) a solid (V) comprising caprolactam is then obtained from the mixture (IV) by crystallization, the proportion by weight of caprolactam in the solid (V) being greater than in the mixture (IV) and the crystallization being effected in the absence of organic or inorganic liquid diluents.

**2.** The process according to claim 1 wherein the mixture (I) additionally comprises an organic liquid diluent.

**3.** The process according to claim 2 wherein the liquid diluent is removed in step c) before, during or after the separation of the water from the mixture (III).

**4.** The process according to any of claims 1 to 3 wherein the crystallization in step d) is effected on a cooled surface on which the solid (V) grows.

**5.** The process according to any of claims 1 to 4 wherein the mother liquor obtained after the crystallization of step d) is mixed with the mixture (IV) and recycled into step d).

**6.** The process according to any of claims 1 to 5 wherein the crystallization in step d) is effected batchwise.

**Revendications**

**1.** Procédé pour la préparation de caprolactame, **caractérisé en ce que**

a) on transforme un mélange (I) contenant du 6-aminocapronitrile et de l'eau, en phase liquide, en un mélange (II) contenant du caprolactame, de l'ammoniac, de l'eau, des substances à point d'ébullition élevé et des substances à point d'ébullition bas en présence d'un catalyseur, ensuite
b) on élimine l'ammoniac du mélange (II) avec obtention d'un mélange (III) contenant du caprolactame, de l'eau, des substances à point d'ébullition élevé et des substances à point d'ébullition bas, puis
c) on élimine l'eau du mélange (III) avec obtention d'un mélange (IV) contenant du caprolactame, des substances à point d'ébullition élevé présentant un point d'ébullition supérieur à celui du caprolactame et des substances à point d'ébullition bas présentant un point d'ébullition inférieur à celui du caprolactame, et on sépare entre les étapes c) et d) les substances à point d'ébullition bas et les substances à point d'ébullition élevé par distillation, puis
d) on obtient à partir du mélange (IV), par cristallisation, un solide (V) contenant du caprolactame, la proportion pondérale de caprolactame dans le solide (V) étant supérieure à celle dans le mélange (IV) et la cristallisation étant réalisée en l'absence de diluants liquides organiques ou inorganiques.

2.  Procédé selon la revendication 1, où le mélange (I) contient en outre un diluant liquide organique.

3.  Procédé selon la revendication 2, où on élimine le diluant liquide dans l'étape c) avant, pendant ou après la séparation de l'eau du mélange (III).

4.  Procédé selon les revendications 1 à 3, où on réalise la cristallisation dans l'étape d) sur une surface refroidie sur laquelle le solide (V) croît.

5.  Procédé selon les revendications 1 à 4, où on mélange la lessive-mère obtenue après la cristallisation selon l'étape d) avec le mélange (IV) et on recycle le mélange dans l'étape d).

6.  Procédé selon les revendications 1 à 5, où on réalise la cristallisation dans l'étape d) de manière discontinue.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1253716 A
- DD 75083 A
- EP 411455 A
- WO 9702228 A
- WO 9706126 A
- WO 9948867 A
- WO 9837063 A
- WO 9965873 A
- US 496941 A
- US 5496941 A
- WO 9620923 A
- US 4601859 A
- US 2762835 A

- WO 9221650 A
- US 2208598 A
- DE 954416 B
- US 2257814 A
- DE 4235466 A
- US 2245129 A
- US 2301964 A
- EP 150295 A
- FR 2029540 A
- US 5646277 A
- US 5739324 A
- WO 5914665 A
- WO 9514664 A

**In der Beschreibung aufgeführte Nicht-Patentliteratur**

- Ullmann's Encyclopedia of Industrial Chemistry. VCH Verlagsgesellschaft mbH, 1986, vol. A5, 46-48
- **Kirk-Othmer.** Encyclopedia of Chemical Technology. John Wiley & Sons, 1992, vol. 4, 836

- Ullmann's Encyclopedia of Industrial Chemistry. VCH Verlagsgesellschaft mbH, 1986, vol. A5, 46
- **Kirk-Othmer.** Encyclopedia of Chemical Technology. John Wiley & Sons, 1979, vol. 7, 870-881